(19) Europäisches Patentamt
European Patent Office
Office européen des brevets

(11) **EP 3 150 583 B1**

(12) **EUROPÄISCHE PATENTSCHRIFT**

(45) Veröffentlichungstag und Bekanntmachung des Hinweises auf die Patenterteilung:
**22.05.2019 Patentblatt 2019/21**

(51) Int Cl.:
***C07D 229/00*** *(2006.01)*

(21) Anmeldenummer: **16197899.4**

(22) Anmeldetag: **13.11.2012**

(54) **MDI-DIMER**

MDI DIMER

DIMÈRE MDI

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**05.04.2017 Patentblatt 2017/14**

(62) Dokumentnummer(n) der früheren Anmeldung(en) nach Art. 76 EPÜ:
**12192416.1 / 2 730 563**

(73) Patentinhaber: **EMS-PATENT AG**
**7013 Domat / Ems (CH)**

(72) Erfinder: **Kaplan, Andreas**
**7013 Domat/Ems (CH)**

(74) Vertreter: **Pfenning, Meinig & Partner mbB**
**Patent- und Rechtsanwälte**
**Theresienhöhe 11a**
**80339 München (DE)**

(56) Entgegenhaltungen:
**EP-A1- 0 195 917     EP-A1- 0 572 994**
**DE-A1- 1 643 170     JP-A- 9 328 474**
**US-A- 5 565 527**

Bemerkungen:
Die Akte enthält technische Angaben, die nach dem Eingang der Anmeldung eingereicht wurden und die nicht in dieser Patentschrift enthalten sind.

**Beschreibung**

Die Erfindung betrifft ein 4,4'-Methylen-bis(phenylisocyanat)-Dimer (MDI-Dimer), das in hoher Reinheit vorliegt. Das MDI-Dimer zeichnet sich dadurch aus, dass es im Wesentlichen frei von MDI sowie von Harnstoffderivaten ist. Die Erfindung betrifft weiterhin dessen Verwendung als Vernetzer für Polyurethane.

4,4'-Methylenbis(phenylisocyanat)-Dimere (MDI-Dimer) sind bekannt. Das 4,4'-MDI-Dimer besitzt die CAS-Nr. 17589-24-1.

Aus der JP 09-328 474 der Nippon Polyurethane ist bereits ein Verfahren zur Herstellung eines MDI-Dimers bekannt. Bei diesem Verfahren wird zuerst das MDI in Ethylacetat gelöst und mit einem Katalysator versetzt. Das MDI-Dimer wird dann nach 24 Stunden abfiltriert. Wie aus der JP 09-328 474 hervorgeht (siehe Example 3) besteht aber das Reaktionsprodukt nur zu 81 % aus dem MDI-Dimer. Das nach dem japanischen Dokument erhaltene MDI-Dimer weist somit noch hohe Anteile an MDI-Monomer bzw. Harnstoffderivate auf. Derartige MDI-Dimere sind deshalb auch als Vernetzer für Polymere nur bedingt geeignet.

Für viele Anwendungen, insbesondere für die Anwendung als Vernetzer für Polymere ist es aber wichtig, dass das 4,4-MDI-Dimer in hoher Reinheit vorliegt. Insbesondere für die Anwendung als Vernetzer für Polyurethane besteht das Erfordernis, ein 4,4-MDI-Dimer zur Verfügung zu stellen, das frei von MDI-Monomer und Harnstoffderivaten ist. Für derartige Anwendungen ist es auch wichtig, dass das MDI-Dimer gut lagerbar ist und in einer einheitlichen Korngröße zur Verfügung steht.

Ausgehend hiervon ist es die Aufgabe der vorliegenden Erfindung ein 4,4-MDI-Dimer mit hoher Reinheit zur Verfügung zu stellen. Angestrebt wird dabei, dass das 4,4-MDI-Dimer frei von MDI-Monomer und von Harnstoffderivaten ist. Die Erfindung umfasst ein entsprechendes 4,4-MDI-Dimer sowie die Verwendung des 4,4-MDI-Dimer als Vernetzer für Polyurethane.

Die Erfindung wird im Bezug auf das MDI-Dimer durch die Merkmale des Anspruches 1 gelöst. Die Unteransprüche 2 bis 4 zeigen vorteilhafte Weiterbildungen auf. In Anspruch 5 werden erfindungsgemäße Verwendungen genannt.

Die Erfindung betrifft ein 4,4'-Methylenbis(phenylisocyanat)-Dimer. Das MDI-Dimer nach der Erfindung zeichnet sich dadurch aus, dass es weniger als 1 Gew.-%, bevorzugt weniger als 0,5 Gew.-%, besonders bevorzugt weniger als 0,1 Gew.-%, ganz besonders bevorzugt weniger als 0,08 Gew.-% monomeres MDI enthält, in Pulverform vorliegt und der mittlere Durchmesser $d_{50}$ der Granulate des Pulvers auf einen Bereich zwischen 1 und 4 $\mu$m eingestellt ist.

Das MDI-Dimer nach der Erfindung weist bevorzugt zudem keine mit HPLC-MS (High Performance Liquid Chromatography-Massenspektrometrie-Kombination) mit UV-Detektor und Massendetektor nachweisbaren Anteile an Produkten wie Trimere, Tetramere, Pentamere oder noch höhere Oligomere auf. Das erfindungsgemäße MDI-Dimer ist auch bevorzugt im Wesentlichen frei von Harnstoffderivaten. Unter im Wesentlichen frei von Harnstoffderivaten wird verstanden, dass das MDI-Dimer weniger als 10 Gew.-%, bevorzugt weniger als 7 Gew.-%, besonders bevorzugt weniger als 4 Gew.-%, ganz besonders bevorzugt weniger als 2 Gew.-% Harnstoffderivate enthält.

Das zur Herstellung der erfindungsgemäßen MDI-Dimere eingesetzte Verfahren zeichnet sich dadurch aus, dass in einem ersten Verfahrensschritt a) ein Lösungsmittel, das bereits einen Katalysator enthält, bereit gestellt wird, wobei das Lösungsmittel derart ausgewählt ist, dass es als Lösungsmittel für 4,4'-Methylenbis(phenylisocyanat) geeignet ist. Bei diesem ersten Verfahrensschritt ist es zudem erforderlich, dass in einer Inertgasatmosphäre bei möglichst geringen Temperaturen, nämlich bei > 25°C bis max. 45°C gearbeitet wird.

Im nächsten Verfahrensschritt b) wird geschmolzenes und/oder gelöstes 4,4-Methylenbis(phenylisocyanat), d.h. MDI unter Rühren zugegeben. Im Folgenden beginnt dann das Produkt auszufallen.

Anschließend wird dann die Reaktion durch Zugabe eines Desaktivators abgebrochen (Verfahrensschritt c)). Bei diesem Verfahrensschritt ist es entscheidend, dass nach dem Abstoppen der Reaktion noch eine gewisse Zeitspanne weitergerührt wird. Diese Zeitspanne liegt im Bereich von 0,5 bis 1,5 Stunden.

In Anschluss an diesen Verfahrensschritt wird das MDI-Dimer vom Lösungsmittel getrennt (Verfahrensschritt d)) und im Verfahrensschritt e) gereinigt.

Beim beschriebenen Verfahren ist es dabei besonders wichtig, dass die genaue Abfolge der vorstehend beschriebenen Verfahrensschritte a) bis e) exakt eingehalten wird. Besondere Bedeutung kommt dabei der Reaktionsführung zu, insbesondere im Hinblick auf das Temperaturniveau. Es ist wesentlich, dass wie im Verfahrensschritt a) angegeben, eine Temperatur eingehalten wird, die im Bereich > 25°C bis 45°C liegt.

Bevorzugt ist es dabei, wenn die Temperatur im Verfahrensschritt a) auf 30 bis 40°C gehalten wird.

Für die Zugabe des geschmolzenen und/oder gelösten MDI ist ein Zutropfen bevorzugt.

Die Bereitstellung des den Katalysator enthaltenden Lösungsmittels erfolgt unter Inertgasatmosphäre wobei das Inertgas ausgewählt ist aus Stickstoff, Argon und/oder Helium. Bevorzugt ist dabei der Wassergehalt dieser Inertgase auf max. 0,01 Vol.-%, bevorzugt auf max. 0,005 Vol.-% eingestellt.

Geeignete Katalysatoren die in der Lösung nach Verfahrensschritt a) enthalten sind, sind ausgewählt aus tertiären Phosphinen, aminosubstituierten Phosphinen, Imidazolen, Guanidinen, in 3- oder 4-Position substituierte Pyridinen, in 3- und 4-Position substituierte Pyridinen, cyclischen Amidinen, Antimonpentafluorid, Bortrifluorid oder Mischungen davon.

Bevorzugt werden als Katalysatoren tertiäre, aliphatische oder gemischt aliphatisch-aromatische Phosphine, Trialkylphosphine, Tris(N,N-dialkylamino)phosphine, Dialkylimidazole, 4-N,N-Dialkylaminopyridine, Pyridine, die in 3- und 4-Position durch N-Atome substituiert sind, welche über Kohlenstoffsegmente - bevorzugt zweigliedrige, gesättigte Kohlenstoffsegmente - verbunden sind oder Mischungen davon eingesetzt.

Besonders bevorzugt werden als Katalysatoren Tris(N,N-dialkylamino)phosphine, 1,2- Dialkylimidazole, 4-N,N-Dialkylaminopyridine oder Mischungen davon eingesetzt.

Ganz besonders bevorzugt werden als Katalysatoren eingesetzt Tris(N,N-dimethylamino)phosphin, Tris(N,N-diethylamino)phosphin, 4-N,N-Dimethylaminopyridin, 4-N,N-Diethylaminopyridin, 1,2-Dimethylimidazol, 1,2-Diethylimidazol oder Mischungen davon.

Phosphine werden auch als Phosphane oder Phosphorane bezeichnet.

Als Desaktivator für die Abstoppung der Reaktion (Verfahrensschritt c)) können alle an und für sich gängigen Desaktivatoren verwendet werden. Beispiele hierfür sind starke Säuren, Säurechloride, Säureanhydride oder Alkylierungsmittel.

Bevorzugt werden als Desaktivator verwendet Chloressigsäure, Trichloressigsäure, Trifluoressigsäure, Methansulfonsäure, Perfluorbutansulfonsäure, Phosphorsäure, Chlorameisensäure, Benzoylchlorid, Dimethylcarbamidsäurechlorid, Essigsäureanhydrid, Bernsteinsäureanhydrid, Dimethylsulfat, Methyljodid, Toloulsulfonsäuremethylester oder Mischungen davon.

Besonders bevorzugt wird als Desaktivator Benzoylchlorid verwendet.

Die Abtrennung des MDI-Dimers vom Lösungsmittel (Verfahrensschritt d)) erfolgt mit einem oder mit einer Kombination aus mehreren mechanischen Trennverfahren zur Fest-Flüssig-Trennung. Bevorzugt wird das mindestens eine mechanische Trennverfahren ausgewählt aus der Gruppe bestehend aus Sedimentation, Filtration, Zentrifugation und das Abscheiden mit Zyklonen.

Bei der Sedimentation kann die Trennung mittels Gravitation oder Zentrifugalkraft erfolgen.

Bei der Filtration kann die Trennung durch Gravitation, Zentrifugalkraft oder Druckdifferenzen erfolgen. Bei den Druckdifferenzen kann es sich um Unterdruck (Vakuum) oder Überdruck handeln.

Die Filtration kann mittels Nutschen, Scheibenfilter, Trommelfilter, Blattfilter, Plattenfilter, Bandfilter, Scherspaltfilter, Filterpressen, Membranfilterpressen, Bandpressen oder Kombinationen davon erfolgen.

Die Zentrifugation kann mittels Vollmantel-Schneckenzentrifugen, Tellerzentrifugen, Scheibenzentrifugen, Schälzentrifugen, Schubzentrifugen, Becherzentrifugen, Dekantierzentrifugen, Separatoren oder Kombinationen davon erfolgen.

Beim Abscheiden mit Zyklonen werden bevorzugt Hydrozyklone verwendet.

Anschließend an den Verfahrensschritt e) kann wie auch im Prinzip aus dem Stand der Technik schon bekannt, eine Trocknung des MDI-Dimer erfolgen, die bevorzugt bei 40°C bis 60°C im Vakuum oder Inertgasstrom für 12 bis 24 Stunden durchgeführt wird. Besonders bevorzugt erfolgt die Trocknung bei 40 °C bis 50 °C im Vakuum für 12 bis 20 Stunden.

Bevorzugt wird das MDI-Dimer nach dem Verfahrensschritt e) getrocknet.

Lösungsmittel die für das beschriebene Verfahren geeignet sind, sind Lösungsmittel, die in der Lage sind, das MDI-Monomer bei den angegebenen Reaktionstemperaturen, d.h. bei > 25°C bis 45°C vollständig zu lösen. Der Wassergehalt des Lösungsmittels darf maximal 0,04 Gew.-%, bevorzugt maximal 0,02 Gew.-%, besonders bevorzugt maximal 0,01 Gew.-% betragen.

Beispiele für Lösungsmittel sind Aceton, N-Methylpyrrolidon, Benzol, Ethylacetat, Acetonitril, Nitromethan, Kerosin, Octan oder Mischungen hiervon.

Die Erfinder konnten dabei zeigen, dass als Lösungsmittel Ethylacetat besonders geeignet ist. Beim beschriebenen Verfahren wird bevorzugt bei der Reinigung des MDI-Dimers mit dem gleichen Lösungsmittel gearbeitet, dass auch für die Reaktion verwendet wird. Bevorzugt wird dabei somit sowohl für das MDI-Monomer wie auch für den Reinigungsschritt Ethylacetat verwendet.

Das hochreine erfindungsgemäße MDI-Dimer nach der Erfindung, wird in einer Pulverform zur Verfügung gestellt. Dazu wird das MDI-Dimer nach dem Verfahrensschritt d) (Abtrennen des erhaltenen MDI-Dimer vom Lösungsmittel) und der Reinigung des erhaltenen MDI-Dimer mit den Lösungsmittel (Verfahrensschritt e)) noch einem Mahlprozess unterzogen werden, sodass dann eine mittlere Granulatgröße $d_{50}$ der Pulver vorliegt, die im Bereich zwischen 1 und 4 $\mu$m, bevorzugt bei 2 $\mu$m liegt.

Das hochreine MDI-Dimer nach der Erfindung ist besonders geeignet als Vernetzer für Polymere und hier insbesondere für Polyurethane zur Herstellung von Schaum- und Dämmstoffen, z.B. als Weich-, Hart- oder Integralschaum, oder von ungeschäumten festen oder gummielastischen Werkstoffen für technische Artikel, wie z.B. Sitzmöbel, Gießharze, Lackharze, Dichtstoffe, Klebeharze oder Beschichtungsmassen für Kunstleder und Textilhilfsmittel. Es hat sich gezeigt, dass das erfindungsgemäße MDI-Dimer eine äußerst große Reaktionsfähigkeit aufweist und dann zu Endprodukten führt, die nach der Vernetzung exzellente mechanische Eigenschaften aufweisen.

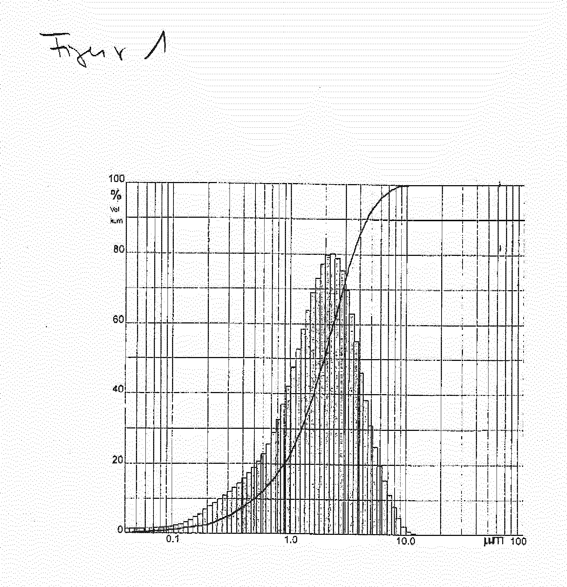

Die Erfindung wird nachfolgend anhand eines allgemeinen Ausführungsbeispiels und eines speziellen Herstellungsbeispiels sowie anhand der Figur 1 näher erläutert.

Figur 1 zeigt die Partikelgrößenverteilung eines erfindungsgemäßen MDI-Dimers, dass nach dem Verfahrensschritt e) noch einer Mahlung unterzogen worden ist. Wie aus Figur 1 hervorgeht, weist das MDI-Dimer in diesem Falle eine sehr enge Größenverteilung auf. Ein MDI-Dimer in einer wie vorstehend beschriebenen hochreinen Form in Kombination mit der sehr feinen Partikelverteilung eignet sich besonders gut als Vernetzer für Polyurethane. Es wurde dabei eine sehr hohe Reaktivität festgestellt, die einerseits auf die hohe Reinheit und andererseits auf die Partikelgrößenverteilung zurückgeführt wird. Weiterhin ist ein MDI-Dimer wie vorstehend beschrieben ausgezeichnet lagerfähig und deshalb besonders gut geeignet als Vernetzer für Polyurethane.

Die Herstellung des MDI-Dimers erfolgt in Lösung in einer inerten Atmosphäre unter Wasserausschluss bei 30 bis 40 °C. Das Lösungsmittel enthaltend den gelösten Katalysator wird vorgelegt. Dazu wird unter Rühren geschmolzenes oder gelöstes MDI innerhalb von 0,5 bis 3 h zugetropft. Nach etwa der Hälfte der Zugabe beginnt das Produkt auszufallen. Nach dem Zutropfen wird noch 1 bis 4 h bei der Reaktionstemperatur gerührt. Anschliessend wird die Reaktion durch Zugabe des Desaktivators abgebrochen, wobei nach der Zugabe noch weitere 0,5 bis 1,5 h bei der Reaktionstemperatur gerührt wird. Der Ansatz wird nach dem Abkühlen auf Raumtemperatur durch eine Glas- oder Keramikfritte im Vakuum filtriert und anschliessend mindestens zweimal mit Lösungsmittel gewaschen. Die Trocknung findet im Vakuum oder Inertgasstrom bei 40 bis 60 °C für 12 bis 24 h statt.

Das so hergestellte MDI-Dimer enthält weder Trimere, Tetramere, Pentamere noch höhere Oligomere, welche mittels HPLC-MS mit UV-Detektor und Massendetektor nachweisbar sind.

Als Inertgase werden Stickstoff, Argon oder Helium verwendet, deren Wassergehalt maximal 0,01 Vol.-% beträgt.

Das Lösungsmittel wird auf einen Wassergehalt von maximal 0,04 Gew.-% getrocknet.

Sollte für die Reaktion geschmolzenes MDI verwendet werden, wird es bei maximal 60 °C, bevorzugt bei 45 bis 55 °C aufgeschmolzen.

**Bestimmung des Gehalts an monomerem MDI im MDI-Dimer**

Das monomere MDI wird aus dem MDI-Dimer extrahiert und im Extrakt mit Ethanol derivatisiert. Das MDI-Dimer wird dazu mit 25 ml Ethylacetat in einem 50 ml Rundkolben fünf Stunden bei 23 °C extrahiert, wobei mit einem Magnetrührer gerührt wird. Der Extrakt wird filtriert und das Filtrat in einen 50 ml-Messkolben überführt. Anschliessend wird der Extrakt mit Ethanol auf 50 ml aufgefüllt, bei 23 °C 30 min stehen gelassen, filtriert und in ein Glasfläschchen abgefüllt. Dieser Vorgang wird mit einer niedrigen Einwaage (1 mg MDI-Dimer pro ml Ethylacetat) und mit einer hohen Einwaage (25 mg MDI-Dimer pro ml Ethylacetat) durchgeführt.

Die Bestimmung der Konzentration an monomerem MDI im Extrakt erfolgt mit HPLC (High Performance Liquid Chromatography) nach der Methode des externen Standards. Als HPLC Gerät wird dabei ein Acquity UPLC der Firma Waters Corp. verwendet, als Säule BEH C18 von Waters Corp. (10,0 cm lang, Innendurchmesser 2,1 mm, Partikelgrösse der Füllung 1,7 $\mu$m). Als Eluent dient eine Acetonitril/Wasser-Gradienten-Mischung mit einer Flussrate von 0,4 ml/min. Die Detektion erfolgt mit einem UV-Detektor bei einer Wellenlänge von 254 nm. Als externer Standard wird MDI verwendet. Mindestens 3 Einwaagen werden eingewogen, in 50mL Ethylacetat/Ethanol 1/1 (vol/vol) gelöst und abgefüllt. Die Kalibriergerade wird mit der Software Empower 2 von Waters Corp. erstellt und muss einen Korrelationskoeffizienten von mindestens 0.999 aufweisen.

Der Gehalt des monomeren MDI in Gew.-% wird nach der folgenden Formel berechnet:

$$\text{monomeres MDI} \; = \; \left([(KA - KB) \times V] : EA\right) \times 100$$

KA     Konzentration des monomeren MDI in mg/ml bei hoher Einwaage
KB     Konzentration des monomeren MDI in mg/ml bei niedriger Einwaage
V      Volumen Ethylacetat in ml
EA     Einwaage des MDI-Dimeren bei hoher Einwaage in mg

Acetonitril, Ethanol und Eythylacetat werden in der Reinheit p.a. (zur Analyse) verwendet. Bei Wasser handelt es sich um bidestilliertes Wasser.

Angegeben wird der arithmetische Mittelwert aus drei Messungen.

**Bestimmung der Reinheit des MDI-Dimeren**

Die Reinheit des MDI-Dimeren wird mit HPLC-MS (High Performance Liquid Chromatography-Massenspektrometrie-Kombination) mit UV-Detektor und Massendetektor bestimmt, wobei die Zuordnung der Peaks über die Masse erfolgt. Die Reinheit des MDI-Dimeren wird in Flächen-% (Fl.-%) angegeben, die Gesamtfläche aller Peaks entspricht dabei 100 Fl.-%. Die angegebenen Werte sind der arithmetische Mittelwert aus drei Messungen.

10 mg des MDI-Dimeren werden dazu in einer Mischung aus 5 ml Tetrahydrofuran und 5 ml Methanol bei 23 °C gelöst, zur Derivatisierung mit dem Methanol danach noch 30 min stehen gelassen, filtriert und in ein Glasfläschchen abgefüllt.

Als HPLC Gerät wird ein Acquity UPLC der Firma Waters Corp. verwendet, als Säule BEH C18 der Waters Corp. (10,0 cm lang, Innendurchmesser 2,1 mm, Partikelgrösse der Füllung 1,7 $\mu$m). Als Eluent dient eine Acetonitril/Wasser-Gradienten-Mischung. Der UV-Detektor arbeitet bei 254 nm und der Massendetektor SQ im ESI-Modus (Elektro-Spray-Ionisations-Modus).

Acetonitril, Methanol und Tetrahydrofuran werden in der Reinheit p.a. (zur Analyse) verwendet. Bei Wasser handelt es sich um bidestilliertes Wasser.

**Herstellungsbeispiele für 4,4'-Methylenbis(phenylisocyanat)-Dimer**

Die Reaktionsapparatur besteht aus einem doppelwandigen, zylindrischem Reaktionsgefäss (Volumen 11) mit Bodenablassventil, Rührer, Schliffdeckel, drei Tropftrichtern, zwei davon doppelwandig, einer mit dem Bodenablassventil verbundenen G4-Fritte und einer Vakuumpumpe.

Die Apparatur wird dreimal im Wechsel von Vakuum (30 mbar) und Stickstoff inertisiert. Die gesamte Herstellung des MDI-Dimers findet unter einem Stickstoffstrom statt.

Im auf 35 °C thermostatisierten Reaktiongefäss werden mittels eines Tropftrichters 500 ml Ethylacetat in dem 1,0 g 4-N,N-Dimethylaminopyridin gelöst ist vorgelegt und im Reaktionsgefäss auf 35 °C thermostatisiert.

130,0 g im Umluftofen bei 50 °C aufgeschmolzenes MDI werden über einen auf 50 °C thermostatisierten Tropftrichter in 45 Minuten unter Rühren zugegeben. Dabei wird darauf geachtet, dass die Temperatur des Reaktionsgemisches bei 35 ° C bleibt. Nach etwa der Hälfte der Zugabe beginnt das Produkt auszufallen.

Im Anschluss an die MDI-Zugabe wird noch 2 Stunden bei 35 °C gerührt.

Danach werden 1,27 g Benzoylchlorid gelöst in 5 ml Ethylacetat über einen auf 35 °C thermostatisierten Tropftrichter in 10 Minuten unter Rühren zugegeben und weitere 15 min gerührt.

Anschliessend wird der Ansatz auf 23 °C abgekühlt und durch die G4-Fritte bei 550 mbar filtriert. Das Produkt wird zweimal mit je 100 ml Ethylacetat gewaschen und bei 50 °C und 30 mbar 16 h getrocknet.

Ausbeute: 120,5 g, d.h. 92,7 Gew.-% bezogen auf die MDI-Einwaage.

Das Produkt enthält:

| | | |
|---|---|---|
| 0,07 | Gew.-% | monomeres MDI* |
| 99,5 | Fl.-% | MDI-Dimer, derivatisiert** |
| 0,5 | Fl.-% | Harnstoff-Dimer, derivatisiert** |

\* bestimmt durch Extraktion und anschliessender HPLC
\*\* bestimmt durch HPLC-MS, angegeben in Flächen-% (Fl.-%)

Weiterhin wird ein Verfahren gemäß der Aspekte 1 bis 9 beschrieben. Die Erfindung zeichnet sich zudem durch die folgenden Aspekte10 bis 14 aus:

1. Verfahren zur Herstellung von 4,4'-Methylenbis(phenylisocyanat)-Dimer (MDI-Dimer) mit folgenden Schritten:

a) Bereitstellen eines einen Katalysator enthaltenen Lösungsmittel für 4,4'-Methylenbis(phenylsiocyanat) (MDI) mit einer Inertgasatmosphäre bei > 25°C bis 45°C,
b) Zugabe eines geschmolzenen und/oder gelösten MDI unter Rühren über eine Zeitspanne von 0,5 bis 3 Stunden,
c) Zugabe eines Desaktivators, mit der Maßgabe, dass noch über eine Zeitspanne von 0,5 bis 1,5 Stunden bei der gegebenen Reaktionstemperatur gerührt wird,
d) Abtrennen des erhaltenen MDI-Dimer vom Lösungsmittel und
e) Reinigung des erhaltenen MDI-Dimer mit dem Lösungsmittel.

2. Verfahren nach Aspekt 1, dadurch gekennzeichnet, dass in Verfahrensschritt a) eine Temperatur im Bereich von 30°C bis 40°C eingehalten und das geschmolzene und/oder gelöste MDI (Verfahrensschritt b) zugetropft wird.

3. Verfahren nach Aspekt 1 oder 2, dadurch gekennzeichnet, dass das Inertgas ausgewählt ist aus Stickstoff, Argon und/oder Helium, deren Wassergehalt max. 0,01 Vol.-%, insbesondere 0,005 Vol.-% beträgt.

4. Verfahren nach mind. einem der Aspekte 1 bis 3, dadurch gekennzeichnet, dass der Katalysator ausgewählt ist aus der Gruppe bestehend aus tertiären Phosphinen, aminosubstituierten Phosphinen, Imidazolen, Guanidinen, in 3- oder 4-Position substituierten Pyridinen, in 3- und 4-Position substituierten Pyridinen, cyclischen Amidinen, Antimonpentafluorid, Bortrifluorid und Mischungen davon, bevorzugt ausgewählt aus der Gruppe bestehend aus tertiären, aliphatischen oder gemischt aliphatisch-aromatischen Phosphinen, Trialkylphosphinen, Tris(N,N-dialkylamino)phosphinen, Dialkylimidazolen, 4-N,N-Dialkylaminopyridinen, Pyridinen, die in 3- und 4-Position durch N-Atome substituiert sind, welche über Kohlenstoffsegmente, insbesondere zweigliedrige, gesättigte Kohlenstoffsegmente, verbunden sind und Mischungen davon, besonders bevorzugt ausgewählt aus der Gruppe bestehend aus Tris(N,N-dialkylamino)phosphinen, 1,2- Dialkylimidazolen, 4-N,N-Dialkylaminopyridinen und Mischungen davon und ganz besonders bevorzugt ausgewählt aus der Gruppe bestehend aus Tris(N,N-dimethylamino)phosphin, Tris(N,N-diethylamino)phosphin, 4-N,N-Dimethylaminopyridin, 4-N,N-Diethylaminopyridin, 1,2-Dimethylimidazol, 1,2-Diethylimidazol und Mischungen davon.

5. Verfahren nach mind. einem der Aspekte 1 bis 4, dadurch gekennzeichnet, dass der Desaktivator ausgewählt ist aus der Gruppe bestehend aus starken Säuren, Säurechloriden, Säureanhydriden oder Alkylierungsmitteln, bevorzugt ausgewählt aus der Gruppe bestehend aus Chloressigsäure, Trichloressigsäure, Trifluoressigsäure, Methansulfonsäure, Perfluorbutansulfonsäure, Phosphorsäure, Chlorameisensäure, Benzoylchlorid, Dimethylcarbamidsäurechlorid, Essigsäureanhydrid, Bernsteinsäureanhydrid, Dimethylsulfat, Methyljodid, Toloulsulfonsäuremethylester und Mischungen davon und besonders bevorzugt Benzoylchlorid.

6. Verfahren nach Aspekt 1 bis 5, dadurch gekennzeichnet, dass die Abtrennung des Reaktionsproduktes in Verfahrensschritt d) mittels einem oder mittels einer Kombination aus mehreren mechanischen Trennverfahren zur Fest-Flüssig-Trennung erfolgt, wobei die mechanischen Trennverfahren bevorzugt ausgewählt sind aus der Gruppe bestehend aus Sedimentation, Filtration, Zentrifugation und dem Abscheiden mit Zyklonen.

7. Verfahren nach mind. einem der Aspekte 1 bis 6, dadurch gekennzeichnet, dass nach dem Verfahrensschritt e) eine Trocknung, bevorzugt im Vakuum oder Inertgasstrom bei 40°C bis 60°C für 12 bis 24 Stunden durchgeführt wird.

8. Verfahren nach mind. einem der Aspekte 1 bis 7, dadurch gekennzeichnet, dass das Lösungsmittel ausgewählt ist aus der Gruppe bestehend aus Aceton, N-Methylpyrrolidon, Benzol, Ethylacetat, Acetonitril, Nitromethan, Kerosin, Octan und Mischungen hiervon, besonders bevorzugt Ethylacetat.

9. Verfahren nach mind. einem der Aspekte 1 bis 8, dadurch gekennzeichnet, dass der Wassergehalt des Lösungsmittels bevorzugt max. 0,04 Gew.-%, besonders bevorzugt maximal 0,02 Gew.-% und ganz besonders bevorzugt 0,01 Gew.-% beträgt.

10. 4,4'-Methylenbis(phenylisocyanat)-Dimer (MDI-Dimer) erhältlich mit einem Verfahren nach mind. einem der Aspekte 1 bis 9, dadurch gekennzeichnet, dass das MDI-Dimer weniger als 1 Gew.-%, bevorzugt weniger als 0,5 Gew.-%, besonders bevorzugt weniger als 0,1 Gew.-%, ganz besonders bevorzugt weniger als 0,08 Gew.-% monomeres MDI enthält, welches in Pulverform vorliegt und der mittlere Durchmesser $d_{50}$ der Granulate des Pulvers auf einen Bereich zwischen 1 und 4 $\mu$m eingestellt ist.

11. MDI-Dimer nach Aspekt 10, dadurch gekennzeichnet, dass es keine oligomeren Produkte wie Trimere, Tetramere, Pentamere oder noch höhere Oligomere enthält.

12. MDI-Dimer nach Aspekt 10 oder 11, dadurch gekennzeichnet, dass es im Wesentlichen frei von Harnstoffderivaten ist, insbesondere weniger als 10 Gew.-%, bevorzugt weniger als 7 Gew.-%, besonders bevorzugt weniger als 4 Gew.-% und ganz besonders bevorzugt weniger als 2 Gew.-%.

13. MDI-Dimer nach mind. einem der Aspekte 10 bis 12, dadurch gekennzeichnet, dass der mittlere Durchmesser $d_{50}$ der Granulate des Pulvers auf auf 2 $\mu$m eingestellt wird.

14. Verwendung eines MDI-Dimer nach mind. einem der Aspekte 10 bis 13 als Vernetzer für Polymere, insbesondere für Polyurethane zur Herstellung von Schaum- und Dämmstoffen, z.B. als Weich-, Hart- oder Integralschaum, oder von ungeschäumten festen oder gummielastischen Werkstoffen für technische Artikel, wie z.B. Sitzmöbel, Gießharze, Lackharze, Dichtstoffe, Klebeharze oder Beschichtungsmassen für Kunstleder und Textilhilfsmittel.

**Patentansprüche**

1. 4,4'-Methylenbis(phenylisocyanat)-Dimer (MDI-Dimer), **dadurch gekennzeichnet, dass** das MDI-Dimer weniger als 1 Gew.-%, bevorzugt weniger als 0,5 Gew.-%, besonders bevorzugt weniger als 0,1 Gew.-%, ganz besonders bevorzugt weniger als 0,08 Gew. % monomeres MDI enthält,
in Pulverform vorliegt und

der mittlere Durchmesser $d_{50}$ der Granulate des Pulvers auf einen Bereich zwischen 1 und 4 $\mu$m eingestellt ist.

2. MDI-Dimer nach Anspruch 1, **dadurch gekennzeichnet, dass** es keine oligomeren Produkte wie Trimere, Tetramere, Pentamere oder noch höhere Oligomere enthält. s

3. MDI-Dimer nach einem der vorhergehenden Ansprüche, **dadurch gekennzeichnet, dass** es im Wesentlichen frei von Harnstoffderivaten ist, insbesondere weniger als 10 Gew.-%, bevorzugt weniger als 7 Gew.-%, besonders bevorzugt weniger als 4 Gew.-% und ganz besonders bevorzugt weniger als 2 Gew.-%.

4. MDI-Dimer nach einem der vorhergehenden Ansprüche, **dadurch gekennzeichnet, dass** der mittlere Durchmesser $d_{50}$ der Granulate des Pulvers auf 2 $\mu$m eingestellt wird.

5. Verwendung eines MDI-Dimer nach einem der vorhergehenden Ansprüche als Vernetzer für Polymere, insbesondere für Polyurethane zur Herstellung von Schaum- und Dämmstoffen, z.B. als Weich-, Hart- oder Integralschaum, oder von ungeschäumten festen oder gummielastischen Werkstoffen für technische Artikel, wie z.B. Sitzmöbel, Gießharze, Lackharze, Dichtstoffe, Klebeharze oder Beschichtungsmassen für Kunstleder und Textilhilfsmittel.

**Claims**

1. 4,4'-methylenebis(phenylisocyanate) dimer (MDI dimer), **characterized in that** the MDI-dimer
   comprises less than 1% by weight, preferably less than 0.5% by weight, particularly preferred less than 0.1% by weight, very particularly preferred less than 0.08% by weight, of monomeric MDI,
   is present in powder form and
   the average diameter $d_{50}$ of the granulates of the powder is adjusted to a range between 1 and 4 $\mu$m.

2. MDI dimer according to claim 1, **characterised in that** it comprises no oligomeric products, such as trimers, tetramers, pentamers or even higher oligomers.

3. MDI dimer according to one of the preceding claims, **characterised in that** it is essentially free of urea derivatives, in particular less than 10% by weight, preferably less than 7% by weight, particularly preferred less than 4% by weight and very particularly preferred less than 2% by weight.

4. MDI dimer according to one of the preceding claims, **characterised in that** the average diameter $d_{50}$ of the granulates of the powder is adjusted to 2 $\mu$m.

5. Use of an MDI dimer according to one of the preceding claims as crosslinker for polymers, in particular for polyurethanes for the production of foam- and insulating materials, e.g. as soft-, hard- or integral foam, or of unfoamed solid or rubber-elastic materials for technical articles, such as e.g. seating, casting resins, paint resins, sealing materials, adhesive resins or coating materials for synthetic leather and textile aids.

**Revendications**

1. Dimère de 4,4'-méthylènebis(isocyanate de phényle) (dimère de MDI), **caractérisé en ce que** le dimère de MDI contient moins de 1 % en poids, de préférence moins de 0,5 % en poids, d'une manière particulièrement préférée moins de 0,1 % en poids, d'une manière tout particulièrement préférée moins 0,08 % en poids de MDI monomère, se présente sous forme de poudre, et
   le diamètre moyen $d_{50}$ des granulés de la poudre est ajusté à une plage comprise entre 1 et 4 $\mu$m.

2. Dimère de MDI selon la revendication 1, **caractérisé en ce qu'**il ne contient aucun produit oligomère tel que des trimères, des tétramères, des pentamères ou encore des oligomères supérieurs.

3. Dimère de MDI selon l'une des revendications précédentes, **caractérisé en ce qu'**il est pour l'essentiel exempt de dérivés de l'urée, et en contient en particulier moins de 10 % en poids, de préférence moins de 7 % en poids, d'une manière particulièrement préférée moins de 4 % en poids et d'une manière tout particulièrement préférée moins de 2 % en poids.

**4.** Dimère de MDI selon l'une des revendications précédentes, **caractérisé en ce que** le diamètre moyen $d_{50}$ des granulés de la poudre est ajusté à 2 $\mu$m.

**5.** Utilisation d'un dimère de MDI selon l'une des revendications précédentes en tant qu'agent de réticulation pour des polymères, en particulier pour des polyuréthannes destinés à la fabrication de mousses et de matériaux isolants, par exemple sous forme d'une mousse souple, d'une mousse dure ou d'une mousse intégrale, ou de matériaux solides ou élastiques non expansés pour articles techniques, tels que par exemple des sièges, des résines de coulée, des résines pour vernis, des matériaux d'étanchéité, des résines adhésives ou des compositions de revêtement pour similicuirs et adjuvants pour textiles.

Figur 1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- JP 9328474 A